Europäisches Patentamt

European Patent Office

Office européen des brevets

(11) Publication number: **0 217 666**
**A2**

# (12) EUROPEAN PATENT APPLICATION

(21) Application number: **86307596.6**

(22) Date of filing: **02.10.86**

(51) Int. Cl.⁴: **A 41 B 13/02**

(30) Priority: **02.10.85 US 782840**

(43) Date of publication of application:
**08.04.87 Bulletin 87/15**

(84) Designated Contracting States:
**AT BE CH DE ES FR GR IT LI LU NL SE**

(71) Applicant: **THE PROCTER & GAMBLE COMPANY**
**One Procter & Gamble Plaza**
**Cincinnati Ohio 45202 (US)**

(72) Inventor: **Lehrer, Steven Bruce**
**9117 Winton Road, No. 5**
**Cincinnati Ohio 45231 (US)**

(74) Representative: **Gibson, Tony Nicholas et al**
**Procter & Gamble (NTC) Limited Whitley Road**
**Longbenton Newcastle upon Tyne NE12 9TS (GB)**

(54) Disposable diaper with superabsorbent strips.

(57) Disposable diapers having spaced apart elongated superabsorbent strips placed between the topsheet and the absorbent core. The superabsorbent strips preferably comprise a laminate of superabsorbent material placed between two sheets of tissue paper. The diaper also includes an impermeable backsheet and, optionally, elements commonly found in disposable diapers, such as leg elastics, waist elastics, fastening tapes, end shields, and the like.

EP 0 217 666 A2

## DISPOSABLE DIAPER WITH SUPERABSORBENT STRIPS

## BACKGROUND OF THE INVENTION

Field of Invention
This invention concerns disposable diapers, incontinent briefs, and the like.

Background Art
Disposable diapers are garments designed to be worn, primarily by infants, about the lower portion of the trunk and to receive discharged urine, feces, and other body fluids. Disposable diapers function to contain the discharged materials and isolate them both from the body of the wearer and from the wearer's surroundings.

For many years diapers were formed from pieces of cloth which were pinned about the lower portion of the wearer's trunk and which were intended to be laundered and reused. In more recent times, disposable diapers have come into vogue thereby freeing mothers (and other charged with the care of infants) from the distasteful task of collecting and laundering soiled cloth diapers. In addition to the logistical benefit provided by disposable diapers, modern embodiments of disposable diapers frequently perform in a manner superior to that of cloth diapers in that they tend to present a drier surface to the body of the wearer and tend to prevent soiling of the wearer's outer garments and other surrounding surfaces more effectively and efficiently than cloth diapers even when the latter are encased within rubber or plastic pants.

Disposable diapers, incontinent briefs, and the like normally comprise three elements: a liquid permeable topsheet intended to be placed next to the wearer's skin; a liquid impermeable backsheet which forms, in use, the outer surface of the diaper, incontinent pad, or the like; and an absorbent core interposed between the topsheet and the backsheet.

The topsheet is frequently a hydrophobic nonwoven fabric. It is readily fluid permeable so that urine will freely pass through it into the absorbent core. Its hydrophobic nature tends to cause its upper surface (i.e., the surface away from the absorbent core and, in use, adjacent the wearer's skin) to be drier and, therefore, protect the wearer's skin from the fluids absorbed within the absorbent element.

The absorbent core is, as its name implies, designed to receive and retain fluids which pass through the topsheet. It normally comprises layers of creped wadding or, more commonly, a batt of airlaid wood pulp fibers.

The backsheet functions to contain body fluids within the absorbent core and to protect the wearer's outer garments and other surfaces from soiling by those fluids. Commonly, the backsheet comprises a fluid impermeable, vapor impermeable material such as polyethylene film.

While many previously known disposable diapers, particularly those mentioned more specifically hereinafter, do perform their tasks well, diapers having improved efficiency in terms of containment of body discharges and topsheet dryness are still being sought. The present invention represents a significant advance in these two last mentioned properties.

## SUMMARY OF THE INVENTION

The present invention is of disposable diapers, incontinent briefs, and the like which present a drier surface to the skin of the wearer and which provide for enhanced efficiency of absorption and containment of body fluids.

The present invention provides a plurality of superabsorbent strips interposed between the topsheet of the diaper and the absorbent core. Preferably, hydrophilic tissue is placed adjacent the topsheet and between the topsheet and the superabsorbent strips.

## BRIEF DESCRIPTION OF THE DRAWINGS

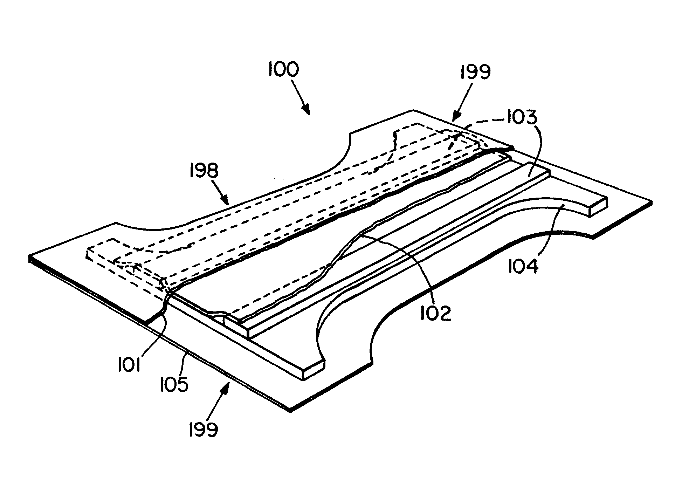

The figure is a simplified, partially cut away perspective view of a disposable diaper of the present invention. The thickness of certain elements has been exaggerated for clarity.

## DETAILED DESCRIPTION OF THE INVENTION

While this specification concludes with claims particularly pointing out and distinctly claiming the subject matter regarded as the invention, it is believed that a better understanding of the invention can be achieved through careful reading of the following detailed description of the invention in conjunction with study of the attached drawing.

The present invention is of a distinct improvement in disposable diapers, incontinent briefs, sanitary napkins, and the like. While it will be described in terms of disposable diapers intended for wear by infants, it is to be understood that the significant advances made by the invention can be applied to a variety of related absorbent devices such as those mentioned.

Disposable diapers have heretofore been presented in a variety of embodiments; it is intended that the present invention be used in conjunction with the various known embodiments. In particular, U.S. Patent Re 26,151, which was issued to Duncan and Baker on January 31, 1967, describes and claims a disposable diaper which has achieved wide acceptance and commercial success. U.S. Patent 3,860,003, which was issued to Buell on January 14, 1975, describes and claims significantly improved disposable diapers which, too, have achieved wide acceptance and commercial success. The diaper taught by Buell differs from that taught by Duncan and Baker in many respects, not the least of which is the provision in the Buell diaper of a contractible leg opening having a side flap which is of sufficient width and flexibility to provide continued non-slipping contact with the wearer's body thereby providing improved containment of fluids.

A still different embodiment of a disposable diaper was described and claimed by Aziz and Blaney in European Patent Publication No. 0070584. The Aziz and Blaney diaper also differs from the Duncan and Baker diaper in many respects, not the least of which is the provision of a multiplicity of flaps which fit about the legs of the wearer when the diaper is worn and which have a fixed edge connected to the outer covering layer and an elasticized distal edge spaced from the fixed edge.

Another form of disposable diaper (sometimes referred to as an incontinent brief and intended to be worn by adults) is shown in U.S. Patent 4,253,461 issued to Strickland and Visscher on March 3, 1981.

Diaper 100 shown in the figure is a simplified representation of a disposable diaper. It illustrates only the most basic elements of a disposable diaper. It is to be understood that additional elements (components) well known to those skilled in the art as commonly being used in disposable diapers (e.g. fastening tapes, leg and waist elastics, waistshields, breathable cuffs, and the like) can be optionally added at the discretion of the manufacturer. For example, fastening tapes are shown in U.S. Patent 3,848,594 issued to Buell on November 19, 1974. Waistshields are shown in Canadian Patent 1,182,601 (issued to Buell on February 19, 1985). Elastic waistbands are shown in U.S. Patent 4,515,595 issued to Kievit and Osterhage on May 7, 1985.

In the figure, disposable diaper 100 is shown as an hourglass-shaped (contoured) disposable diaper. Other shapes, and the precise dimensions of the diaper, can be readily selected by the skilled artisan.

Disposable diaper 100 comprises topsheet 101, absorbent core 104, and backsheet 105. Diaper 100 includes a crotch region 198 which, when diaper 100 is in use, is placed between the legs of the infant adjacent the infant's crotch region. Diaper 100 also has waist regions 199 which, when diaper 100 is in use, are placed adjacent the infant's waist.

Topsheet 101 can be any compliant, soft feeling, non-irritating (to the wearer's skin), liquid permeable, planar material. It can be constructed of porous paper made from natural or synthetic fibers or mixtures thereof, nonwoven fabric made from natural or synthetic fibers or mixtures thereof, apertured plastic film, porous foam, or the like. Examples of suitable topsheets are described, for example, in the hereinbefore incorporated patents of Duncan and Baker and Buell.

A diaper topsheet functions to contact the wearer's skin to receive fluid discharges, to allow the discharges to pass rapidly therethrough into the absorbent core, and to isolate the wearer's skin from the fluids in the absorbent core. To aid in effective performance of the last function, the topsheet is preferably hydrophobic.

A preferred topsheet is spun-bonded nonwoven polyester fabric made from fibers of from 0.24 to 0.27 Tex having a basis weight of 17 grams per square meter. Another preferred topsheet material comprises 65% staple length, 0.16 Tex polyester fibers (such as Kodel type 411 polyester fibers as sold by Tennessee Eastman Corporation, Kingsport, Tennessee); 15% crimped, staple length, 0.16 Tex rayon fibers; and 20% acrylic copolymer binder (such as Celanese CPE 8335, as sold by Celanese Corporation of Charlotte, North Carolina). "Staple length" refers to fibers having a length of at least 15 millimeters.

Suitable topsheets can also be constructed from apertured plastic films such as those described by Radel and Thompson in U.S. Patent 4,342,314, issued August 3, 1982. Another apertured thermoplastic film useful as a topsheet is described with particularity in U.S. Patent 4,341,217 issued to Ferguson and Landrigan on July 27, 1982. A still further suitable topsheet can be formed from a liquid impermeable material provided with tapered capillaries as described in U.S. Patent 3,929,135, issued to Thompson on December 30, 1975.

Another preferred topsheet is constructed from polypropylene fibers which have been carded and thermally bonded in a spaced-apart pattern. Fibers 3.8 centimeters long and of from 0.16 to 0.33 Tex are suitable. A preferred sheet has a basis weight of 22 grams per square meter.

The size and shape of topsheet 101 are, naturally, dictated by the size and shape selected for diaper 100.

As discussed hereinbefore, one function of backsheet 105 is to prevent fluids from escaping from disposable diaper 100 and soiling the wearer's outer garments or other surfaces in contact with the disposable diaper. Any compliant, non-irritating, planar material which is impermeable to body fluid discharges can be used as backsheet 105. Suitable materials are described with particularity in, for example, the hereinbefore mentioned patents to Duncan and Baker and to Buell.

A preferred backsheet is formed from polyethylene film having a thickness of from 0.012 to 0.051 millimeter.

Backsheets which are impermeable to liquid but permeable to vapor are known as breathable backsheets and have been described in the art. Breathable backsheets provide a cooler garment and permit some self-drying of the diaper while it is being worn. As suggested, these breathable backsheets are intended to allow the passage of vapor through them while retarding the passage of liquid. For example, U.S. Patent 3,156,242 issued to Crowe, Jr. on November 10, 1964, teaches the use of a microporous film as a breathable backsheet. U.S. Patent 3,881,489, issued to Hartwell on May 6, 1975, teaches a breathable backsheet comprising, in combination, two layers: a low-void volume perforated thermoplastic film and a porous high-void volume hydrophobic tissue. U.S. Patent 3,989,867, issued to Sisson on November 2, 1976, teaches a breathable backsheet provided with tapered hollowed bosses which prevent the passage of liquids while allowing vapors to pass readily therethrough.

As a general matter, the last two cited patents, provide for breathability across substantially the entire outer surface of the diaper. U.S. Patent 4,341,216, issued to Obenour on July 27, 1982 describes and claims a somewhat different exemplary breathable backsheet. One practical effect of this particular invention is to tend to restrict the vapor permeability of the breathable backsheet in the crotch region relative to the vapor permeability of the backsheet in the waist regions.

The size and shape of backsheet 105 are, naturally, dictated by the size and shape selected for diaper 100.

Absorbent core 104 can be constructed from any of a variety of materials commonly used in disposable absorbent articles and which are described in the hereinbefore mentioned patents. Examples of suitable absorbent materials include creped cellulose wadding, absorbent foams, absorbent sponges, super absorbent polymers, and, preferably, comminuted and airlaid wood pulp commonly referred to as absorbent fluff. When absorbent core 104 comprises absorbent fluff, it usually has a density of from 0.10 to 0.175 grams per cubic centimeter.

The size and shape of absorbent core 104 are, naturally, dictated by the size and shape selected for diaper 100.

Disposable diaper 100 of this invention requires a plurality of superabsorbent strips. In the figure, two such superabsorbent strips 103 are illustrated. While two is the preferred number, more can be used. Superabsorbent strips 103 run generally along the length of absorbent core 104 from one waist region 199 to the other. Superabsorbent strips 103 need not extend to the precise end margin of absorbent core 104, but they should extend over a signficant portion of the length of absorbent core 104. Preferably, superabsorbent strips 103 and absorbent core 104 are coterminus in waist regions 199.

The width of superabsorbent strips 103 depends upon the exact design and size of the particular diaper in question. When the diaper is of hourglass shape as illustrated and is from 10 to 12 centimeters wide in the crotch region, superabsorbent strips 103 are each from 2 to 4 centimeters wide. Each member of the plurality of superabsorbent strips 103 need not be the same width, but they are generally of the same width for economies of manufacture.

Superabsorbent strips 103 must be spaced apart. That is to say, there must be an open, superabsorbent strip-free space between each pair of superabsorbent strips. The distance between each member of any pair of superabsorbent strips need not be equal over the entire length of the superabsorbent strips, but equality of spacing is preferred. Preferably, the members of each pair of superabsorbent strips will be spaced one from another an average distance equivalent to from 0.5 to 1.5 times the width of the wider of the members of the pair.

As shown in the figure, superabsorbent strips 103 are generally planar and are elongated (i.e. the first dimension in the plane is significantly greater than a second dimension taken at right angles to the first). As illustrated, superabsorbent strips 103 are generally rectangular. While this is a preferred configuration, absolute rectangularity is not required.

As noted above, a plurality of superabsorbent strips 103 is required. While two superabsorbent strips are preferred, three, four or more can be used. Since each pair of superabsorbent strips within the plurality must be spaced apart, as noted above, practical considerations of diaper design and manufacturing dictate that only two or three superabsorbent strips are normally considered for use in a diaper intended to be worn by infants. In incontinent briefs intended for use by adults, two, three or four superabsorbent strips can be used. Preferably, however, only two superabsorbent strips will likewise be used in this latter application.

The superabsorbent strips used in the present invention are distinct structural elements of the diaper, which elements are significantly more absorbent per unit volume than is the absorbent core. Preferably, the superabsorbent strips are at least 50% more absorbent per unit volume than is the absorbent core. (As used in this specification, absorbency is intended to refer to the capacity of a given material to take up and retain body fluids such as urine.)

While the superabsorbent strips can be provided by any convenient means well known to those skilled in the art, the superabsorbent strips preferably comprise a quantity of superabsorbent material interposed between two layers of tissue paper. That is to say, the superabsorbent strips are preferably superabsorbent material sandwiches in which tissue paper corresponds to the bread while the superabsorbent material corresponds to the sandwich filling. These "sandwiches" are sometimes referred to as "laminates."

The superabsorbent material can be any of the numerous materials well known to those skilled in the art.

Examples of superabsorbent material include the cross-linked carboxymethyl cellulose disclosed in U.S. Patent No. 3,589,364, issued to Dean and Ferguson on June 29, 1971. Another example is the essentially acidic carboxymethyl cellulose described in U.S. Patent No. 3,678,031, issued to Schoggen on July 18, 1972.

A class of superabsorbent materials which are preferred for use in the present invention includes the water-insoluble hydrogels.

Water-insoluble hydrogels are polymeric materials which are capable of absorbing large quantities of fluids such as urine and which are further capable of retaining such absorbed fluids under moderate pressures.

Suitable hydrogels can be inorganic materials such as silica gels or organic compounds such as cross-linked polymers. Cross-linking may be by covalent, ionic, van der Waals, or hydrogen bonding. Examples of hydrogel polymers include polyacrylamides, polyvinyl alcohol, ethylene maleic anhydride copolymers, polyvinyl ethers, hydroxpropyl cellulose, carboxymethyl cellulose, polyvinyl morpholinone, polymers and copolymers of vinyl sulfonic acid, polyacrylates, polyacrylamides, polyvinyl pyridine and the like. Other suitable hydrogels are those disclosed in U.S. Patent 3,901,236 issued to Assarsson et al on August 26, 1975. Particularly preferred hydrogel polymers for use in the superabsorbent strips are hydrolyzed acrylonitrile grafted starch, acrylic acid grafted starch, polyacrylates, and isobutylene maleic anhydride copolymers, and mixtures thereof.

Processes for preparing hydrogels are disclosed in U.S. Patent 4,076,663, issued to Masuda et al on February 28, 1978; U.S. Patent 4,286,082, issued to Tsubakimoto on August 25, 1981; and further in U.S. Patents 3,734,876, 3,661,815, 3,670,731, 3,664,343, 3,783,871, and Belgian Patent 785,850.

Water-insoluble hydrogels are generally used in the form of discrete particles. Hydrogel particles can be of any desired shape (e.g., spherical or semi-spherical, cubic, rod-like, polyhedral, etc.). Shapes having a large greatest dimension/smallest dimension ratio, like needles, flakes and fibers, are also contemplated for use herein. Conglomerates of hydrogel particles can also be used.

Although the hydrogel-containing superabsorbent strips are expected to perform well with hydrogel particles having a particle size varying over a wide range, other considerations may pre clude the use of very small or very large particles. For reasons of industrial hygiene, average particle sizes smaller than 30 microns are less desirable. Particles having a smallest dimension larger than 2 millimeters may also cause a feeling of grittiness in the disposable diaper, which is undesirable from an aesthetics view. Furthermore, the rate of fluid absorption can be affected by hydrogel particle size with larger particles having reduced rates of absorption. Preferred for use herein are hydrogel particles having an average particle size of from 50 microns to 1 millimeter. "Particle Size" as used herein means the weighted average of the smallest dimension of the individual particles.

The tissue paper used to form the laminate can be any permeable, wet strength paper such as that made by the process described in U.S. Patent 3,301,746 issued to Sanford and Sisson on January 31, 1967.

The superabsorbent strips in the form of a sandwich of tissue paper and superabsorbent material can be constructed by any convenient method. For example, superabsorbent material can be mechanically spread across a sheet of tissue paper at a rate of from 15 to 65 grams per square meter. A second sheet of tissue can be brought in contact with the first with the superabsorbent material interposed between the two sheets of tissue paper. The assembly of two sheets of tissue paper with superabsorbent material interposed therebetween can be subjected to pressure and, optionally, heat and, also optionally, moisture, during the pressure treatment. The thus-formed laminate can then be cut to the desired size and can be used in manufacturing diapers of the present invention.

In highly preferred embodiments of the present invention, a hydrophilic tissue 102 is placed adjacent topsheet 101 and is interposed between topsheet 101 and superabsorbent strips 103. The hydrophobic tissue can be made from any permeable material well known to those skilled in the art. Preferably, the hydrophilic tissue possess wet strength characteristics. Tissues commonly used in disposable diapers as envelope tissues placed about absorbent cores are satisfactory.

Tissue 102 is described as being a "hydrophilic" tissue. This means that the surface of the tissue must be wetted by the fluid in question, be it urine, feces, blood, etc. The state of the art respecting wetting of materials allows definition of hydrophilicity (and wetting) in terms of contact angles and the surface tensions of the fluids and solids involved. This is discussed in detail in the American Chemical Society publication entitled Contact Angle, Wetability, and Adhesion edited by Robert F. Gould, and copyrighted in 1964, which publication is incorporated herein by reference. A surface is said to be wetted by a fluid either when the contact angle between the fluid and the surface is less than 90 or when the fluid will tend to spread spontaneously across the surface; both conditions normally co-exist.

The hydrophilicity of the tissue can be achieved by any convenient means. For example, the material itself can be intrinsically hydrophilic. Or, the tissue can be rendered hydrophilic by treatment with a surfactant, such as a nonionic or an anionic surfactant, as by spraying the tissue with the surfactant or by dipping the tissue into a solution of the surfactant.

The previous discussion concerning topsheet 101 indicated that it was preferably hydrophobic. Diapers using hydrophilic topsheets are not unknown and can benefit from the present invention. In this circumstance (where topsheet 101 is hydrophilic) hydrophilic tissue 102 must be more hydrophilic than the topsheet. Circumstances require this relationship so that fluids deposited on the topsheet will tend to be transported through the topsheet by the differences in hydrophilicity thereby leaving the topsheet drier than it otherwise would be.

In the foregoing description of the diapers, no specific mention has been made of the manner of construction of the diapers. Those skilled in the art will determine that the various elements of the diaper can be assembled into a unified whole as, for example, by securement of the various elements together with hot melt adhesive. Various techniques for uniting the various elements of disposable diapers into a coherent whole are discussed in the hereinbefore incorporated patents.

By way of illustration, and not by way of limitation. the following example is presented.

EXAMPLE

An hourglass-shaped diaper as illustrated in the Figure is constructed from a carded polypropylene non-woven topsheet, an impervious polyethylene film backsheet of 0.025 millimeter thickness, and an absorbent core comprising air laid wood pulp fibers and having a density of 0.11 gram per cubic centimeter. The overall disposable diaper is 44.5 centimeters long; the absorbent core is 39 centimeters long and is 11 centimeters wide in the crotch region. Superabsorbent strips are constructed of a laminate of acrylic acid grafted starch water-insoluble hydrogel interposed between two sheets of wet strength tissue paper. The hydrogel is distributed across the surface of one sheet of tissue paper at a rate of about 40 grams per square meter. The second sheet of tissue paper is placed in contact with the first with the hydrogel interposed therebetween and the unit is subjected to heat and pressure to form a laminate. Two strips of the thus formed superabsorbent-tissue paper laminate, each being 2.5 centimeters wide and 39 centimeters long are placed along the length of the absorbent core as illustrated in the Figure. The two superabsorbent strips are placed parallel and are 2.5 centimeters apart. The two superabsorbent strips are equally spaced from the imaginary longitudinal centerline of the disposable diaper. A sheet of permeable, hydrophilic wet strength tissue paper is interposed between the superabsorbent strips and the topsheet. The entire assembly is held together by lines of hot melt adhesive extending longitudinally along the disposable diaper.

In use, the disposable diaper functions in an exemplary manner and presents to the skin of the wearer a drier topsheet than does an equivalent diaper which does not contain the superabsorbent strips.

**Claims**

1. A disposable diaper comprising a topsheet, a backsheet, and an absorbent core interposed between said topsheet and backsheet characterised in that a plurality of elongated superabsorbent strips is disposed adjacent said topsheet and interposed between said topsheet and said absorbent core, said plurality of superabsorbent strips being oriented generally along the length of said absorbent core, and each of said superabsorbent strips being spaced apart from the adjacent superabsorbent strip.

2. A disposable diaper according to Claim 1 including as an additional element a hydrophilic tissue adjacent said topsheet and interposed between said topsheet and said plurality of superabsorbent strips.

3. A disposable diaper according to either one of claims 1 and 2 wherein the average distance between the members of any pair of said plurality of superabsorbent strips is equivalent to from 0.5 to 1.5 times the width of the wider of said members of said pair.

4. A disposable diaper according to any one of claims 1-3 comprising two superabsorbent strips.